(19) Europäisches Patentamt
European Patent Office
Office européen des brevets

(11) Numéro de publication: **0 187 298**
**B1**

# (12) FASCICULE DE BREVET EUROPEEN

(45) Date de publication du fascicule du brevet: **23.05.90**

(51) Int. Cl.[5]: **C 07 C 219/00**

(21) Numéro de dépôt: **85115808.9**

(22) Date de dépôt: **11.12.85**

## (54) Composés organiques d'ammonium quaternaire et procédé pour leur fabrication.

(30) Priorité: **12.12.84 FR 8419110**

(43) Date de publication de la demande:
**16.07.86 Bulletin 86/29**

(45) Mention de la délivrance du brevet:
**23.05.90 Bulletin 90/21**

(84) Etats contractants désignés:
**BE CH DE FR GB IT LI NL SE**

(56) Documents cités:
**JOURNAL OF THE AMERICAN CHEMICAL SOCIETY, vol. 106, no. 7, 4 avril 1984, pages 1978-1983, American Chemical Society, Wahington D.C., US; T. KUNITAKE et al.: "Bilayer membranes of triple-chain ammonium amphiphiles"**

**BULLETIN OF THE CHEMICAL SOCIETY OF JAPAN, vol. 47, no. 2, février 1974, pages 405-409, Tokyo, JP; T. YOSHINO et al.: "Synthetic studies by the use of carbonates. I. The nucleophilic substitution reaction of ethylene carbonate with amine hydrohalides"**

(73) Titulaire: **INTEROX Société Anonyme
Rue du Prince Albert, 33
B-1050 Bruxelles (BE)**

(72) Inventeur: **Walraevens, René
Avenue des Neuf Provinces, 3
B-1080 Bruxelles (BE)**
Inventeur: **Coisne, Jean-Marc
Boulevard Général Jacques, 182
B-1050 Bruxelles (BE)**

(74) Mandataire: **Lederer, Franz, Dr. et al
Van der Werth, Lederer & Riederer
Patentanwälte Lucile-Grahn-Strasse 22
D-8000 München 80 (DE)**

**EP 0 187 298 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention est relative à des composés organiques d'ammonium quaternaire comprenant plusieurs fonctions esters.

On connaît le chlorure de triméthyle-(2,3-diacétoxypropyl)-ammonium qui est un composé organique d'ammonium quaternaire comprenant deux fonctions ester sur un seul groupe alkyle (Beilstein, vol. IV, 1922 Berlin, B. Prager et P. Jacobson "Aminoderivate der Dioxy-Verbindungen" pages 301 à 303, * page 302, ligne 45 à ligne 48 *).

On a décrit des composés d'ammonium quaternaire estérifiés comportant de longues chaînes alkyle linéaire comprenant plus de 9 atomes de carbone: bromure de méthyl-tris(2-dodécyloxyéthyl)-ammonium et bromure de méthyl-tris(2-hexadécyloxyéthyl)-ammonium (Journal of the American Chemical Society, vol. 106, n° 7, 4 avril 1984, pages 1978-1983, Washington D.C., T. Kunitake et al. "Bilayer membranes of triple-chain ammonium amphiphiles" * page 1, colonne 2 sous 4 *).

On connaît étalement des composés d'ammonium quaternaire comportant 1 groupement ester sur chacun des 4 groupes alkyle liés à l'azote: le tétrakis(2-acétoxyéthyl)-ammonium (Bulletin of the Chemical Society of Japan, vol. 47, n° 2, février 1974, pages 405—409, Tokyo, T. Yoshino et al. "Synthetic Studies by the Use of Carbonates I. The nucleophilic substitution reaction of ethylene carbonate with amine hydrohalides" * page 407, colonne 2, exemple 1 *).

L'objectif de l'invention est de fournir des nouveaux composés organiques comprenant une fonction ammonium quarternaire où au moins trois des groupes liés à l'atome d'azote sont substitués par des groupements esters.

Les composés d'ammonium quaternaire selon l'invention sont des composés de formule générale:

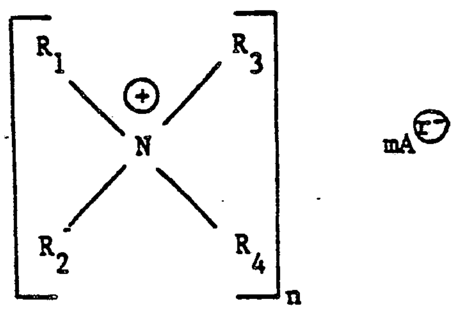

dans laquelle

$R_1$ représente un groupe alkyle, aryle, arylalkyle ou alkylaryle, non substitué ou substitué par au moins deux groupements ester;

$R_2$, $R_3$ et $R_4$ représentent des groupes alkyle, aryle, arylalkyle ou alkylaryle substitués chacun par au moins un groupement ester de formule:

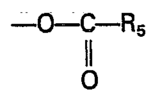

où $R_5$ représente un groupe alkyle, aryle, arylalkyle ou alkylaryle de 1 à 9 atomes de carbone;

$A^{\ominus}$ représente un anion organique ou inorganique;

n et r sont des nombres entiers;

m est un nombre entier valant 1, 2 ou 3 et tel que $m \times r = n$.

Dans les composés selon l'invention, les groupes alkyle sont toutes chaînes aliphatiques ramifiées ou non, tous radicaux cycloalkyles ou toutes chaînes aliphatiques substituées par un radical cycloalkyle éventuellement substitué tels que les chaînes droites ou ramifiées de 1 à 20 atomes de carbone comme les groupes méthyle, propyle, isopropyle, n-butyle, isobutyle, sec-butyle et tert-butyle, n-pentyle, 1-méthylbutyle, 2-méthylbutyle, 3-méthylbutyle, 1-éthylpropyle, 1,1-diméthylpropyle 2,2-diméthylpropyle, n-hexyle et ses isomères ramifiés, n-heptyle, n-octyle, n-nonyle, n-décyle, n-undécyle, n-dodécyle et leurs isomères ramifiés, les groupes cyclopropyle, cyclopentyle et cyclohexyle et les groupes cités comportant une ou plusieurs insaturations.

Les groupes aryle sont tout dérivé aromatique mono ou polycyclique éventuellement substitué tel que les groupes phényle, 2,3-xylyle, 2,4-xylyle, 2,5-xylyle, 2-tolyle, 3-tolyle, 4-tolyle, mésityle, o-cuményle, m-cuményle, p-cuményle, 1-haphtyle, 2-naphtyle, 1-anthryle, 2-anthryle, 1-phénantryle, 2-phénantryle, 2-chrysyle et 2-pyryle.

Les groupes alkylaryle sont tout groupe aryle substitué par un ou plusieurs groupes alkyle saturés ou insaturés tel que les groupes alkylbenzyle, alkylnaphtyle et alkylanthryle.

Les groupes alkylaryle sont tous groupes alkyle substitués par un ou plusieurs groupes aryle tels que les groupes benzyle, phénétyle et trityle.

Par anion inorganique ou organique on entend tout anion provenant d'un acide inorganique ou

organique tel que les anions chlorure, bromure, iodure, fluorure, sulfate, hydrogénosulfate, carbonate, bicarbonate, phosphate, mohohydrogénophosphate, dihydrogénophosphate, pyrophosphate, métaphosphate, thiosulfate, nitrate, méthosulfate, dodécylsulfate, dodécylbenzènesulfonate, phosphonate, méthylphosphonate, methanedisulfonate, méthylsulfonate, éthanesulfonate, 1,2-éthanedisulfonate.

Dans les composés selon l'invention, les groupes $R_2$, $R_3$ et $R_4$ sont substitués chacun par au moins un groupement ester. Celui-ci peut dériver de tout acide mono ou polycarboxylique tel que les acides acétique, propionique, butanoïque, octanoïque, nonanoïque et décanoïque. Des composés qui conviennent bien sont ceux dans lesquels les groupements ester aliphatique sont à chaîne droite ou ramifiée et dont le groupe $R_5$ comprend de 7 à 9 atomes de carbone tels que les chlorures de méthyl-tris(2-octyloxyéthyl)-ammonium. Les deux groupes $R_3$ et $R_4$ peuvent compoter des nombres égaux ou différents de groupements ester. Des composés qui conviennent spécialement bien sont ceux où le groupe $R_5$ contient mons de 7 atomes de carbone et, de préférence, moins de 4 atomes. Ceux dans lesquels le groupe $R_5$ ne contient qu'un atome de carbone sont les plus avantageux.

Des composés conformes à l'invention comprennent, ceux où le groupe $R_1$ n'est pas substitué tels que les sels de méthyl-tris (2-acétoxyéthyl)-ammonium, en particulier le méthosulfate de méthyltris(2-acétoxyéthyl)-ammonium, le dodécylsulfate de méthyl-tris(2-acétoxyéthyl)-ammonium, le dodécylbenzènesulfonate de méthyl-tris(2-acétoxyéthyl)-ammonium et le chlorure de méthyl-tris(2-acétoxyéthyl)-ammonium.

Des composés particulièrement avantageux selon l'invention sont ceux dans lesquels le groupe $R_1$ n'est pas substitué et où l'un au moins des groupes $R_2$, $R_3$ ou $R_4$ comporte deux groupements ester acétique, tel que, par exemple, le chlorure de n-butyl-tris(2,3-diacétoxypropyl)-ammonium.

Les composés préférés selon l'invention sont ceux dans lesquels le groupe $R_1$ est substitué par au moins deux groupements ester, en particulier le chlorure de (2,3-diacétoxypropyl)-tris(2-acétoxyéthyl)-ammonium.

L'invention concerne aussi un procédé de préparation des composés selon l'invention selon lequel on traite une amine tertiaire comprenant un groupement alcool sur chaque groupe lié à l'azote, avec un agent d'alkylation ou d'arylation et avec un agent d'acylation choisi parmi les anhydrides et les chlorures d'acyle.

Dans le procédé selon l'invention, l'amine tertiaire contenant un groupement alcool est toute amine tertiaire dans laquelle les groupes liés directemenet à l'atome d'azote sont des groupes aliphatiques ou aromatiques contenant un groupement alcool. Des exemples d'amines convenant bien sont la triéthanolamine, la tris(2,3-dihydroxypropyl)-amine, la tris(2-hydroxypropyl)-amine, la méthyl-bis(2,3-dihydroxypropyl)-amine, la N,N-diéthanol-aniline. La triéthanolamine est préférée.

L'agent d'alkylation ou d'arylation peut être tout sel d'alkyle, d'alkylaryle ou d'arylalkyle capable de provoquer une quaternisation de l'amine. On peut par exemple le choisir parmi les halogénures et les sulfates d'alkyle, d'aryle et d'alkylaryle comme le sulfate de diméthyle, les chlorure, bromure et iodure de méthyle, d'éthyle ou de benzyle, le sulfate de méthyldodécylbenzène ou pami les halogénohydroxyalkyles ou les halogéno-hydroxyaryles tels que le 2-bromoéthanol, le 1-chloro-2,3 propane-diol, et le 2-chloro-3-phénylpropane-1-ol.

L'agent d'acylation peut être tout composé capable de provoquer une estérification d'au moins deux groupements alcool et est sélectionné parmi les anhydrides et les chlorures d'acyle tels que l'anhydride acétique, l'anhydride benzoïque, l'anhydride phtalique, le chlorure d'acétyle et le chlorure de propionyle.

Dans le procédé selon l'invention, il convient généralement que le traitement avec l'agent d'alkylation et le traitement avec l'agent d'acylation ne soient pas simultanés. Le traitement avec l'agent d'alkylation peut indifféremment précéder ou suivre le traitement avec l'agent d'acylation.

Les composés d'ammonium quaternaire selon l'invention possèdent des propriétés tensio-actives et une aptitude à réagir avec le peroxyde d'hydrogène en milieu aqueux, pour former par perhydrolyse des groupements esters les peracides correspondants. Ils trouvent de ce fait des applications dans l'industrie des détergents où ils constituent des additifs pour les compositions détergentes dans lesquelles ils jouent le rôle d'activants des persels pour le lavage à basse température.

Des particularités de l'invention ressortiront des exemples suivants qui décrivent des procédés de préparation de composés suivant l'invention.

Exemple 1

Préparation du méthosulfate de méthyl-tris(2-acétoxyéthyl)-ammonium

Un mélange de 29,8 g (0.2 mole) de triéthanolamine, 78,5 g (1 mole) de chlorure d'acétyle et 500 ml de chloroforme a été maintenu au reflux pendant 8 heures, période après laquelle le dégagement gazeux d'acide chlorhydrique a cessé.

Après refroidissement, le mélange a été filtré et le filtrat a été évaporé sous une pression de 2,67 kPa à 50°C. Le résidu huileux résultant a ensuite été mélangé avec 25 g d'hydroxyde de sodium en poudre et 250 ml d'éther diéthylique sec après quoi le mélange a été filtré. Le filtrat a alors été évaporé sous une pression de 2,67 kPa à 50°C et le résidu a été distillé sous pression réduite (133 à 200 Pa). La température d'ébullition s'est établie dans ces conditions à 145—146°C. On a recueilli 35,2 g (128 mmoles) de tris (2-acétoxyléthyle)-amine.

On a mélangé la tris(2-acétoxyéthyl)-amine ainsi obtenue avec 17,9 g (142 mmoles) de sulfate de

diméthyle et l'on a maintenu le mélange à 40°C pendant 2 heures. Après refroidissement, le solide formé a été mis en suspension dans 500 ml d'éther diéthylique, filtré et séché à 20°C sous pression de 1,33 kPa.

On a obtenu 51 g de méthosulfate de méthyl-tris (2-acétoxyéthyl)-ammonium (Rendement: 64%).

Exemple 2

Préparation du chlorure de tris(2-acétoxyéthyl)-(2,3 diacétoxypropyl)-ammonium.

*1ère étape*: synthèse du chlorure de tris(2-hydroxyéthyl)-(2,3 dihydroxypropyl)-ammonium.

Dans un ballon surmonté d'un réfrigérant et muni d'un agitateur pneumatique et d'un thermomètre on a introduit 44,8 g de triéthanolamine (0,3 mole) et 33,2 g (0,3 mole) de 1-chloro-2,3-propanediol.

Le mélange a été maintenu pendant 22 heures à 100°C.

On a obtenu un produit brun foncé que l'on a lavé cinq fois par mise en suspension dans 50 ml d'acétone suivie d'agitation, décantation et séparation de la phase acétone.

Après purification dans l'éthanol et séchage sous vide à température ambiante, on a isolé 58,3 g d'un produit brun ayant l'apparence d'un laque. Ce produit a titré 3,51 ion g de $Cl^-$/kg.

*2ème étape*: acétylation du chlorure de tris(2-hydroxyéthyl)-(2,3 dihydroxypropyl)-ammonium.

A 17 g de produit obtenu à la première étape, on a ajouté progressivement pendant 2 heures à température ambiante et sous agitation, 51 g de chlorure d'acétyle. On a observé un important dégagement gazeux d'acide chlorhydrique. On a chauffé ensuite progressivement à 60°C et on a maintenu cette température pendant 5 heures. Le chlorure d'acétyle en excès a ensuite été éliminé par évaporation à 80°C sous pression réduite.

Le résidue d'évaporation s'est présenté sous forme d'une laque très dure et brune. Ce résidu a alors été additoinné de 170 ml de tétrahydrofurane et porté à ébullition pendant 15 min. Après refroidissement, on a séparé la phase tétrahydrofurane puis on a répété l'opération. Le produit s'est présenté sous forme d'un liquide coloré très visqueux. Après élimination du tétrahydrofurane par évaporation sous vide dans un évaporateur rotatif on a obtenu 20,8 g d'une laque incolore très visqueuse.

Le dosage des ions $Cl^-$ a correspondu à 99,7% de la valeur théorique calculée pour la molécule de chlorure de tris (2-acétoxyéthyl)-(2,3 diacétoxypropyl)-ammonium. Le dosage des groupes acétoxy par saponification a correspondu à 98% de la valeur théorique.

Exemple 3

Préparation du chlorure de n-butyl-tris(2,3 diacétoxypropyl)-ammonium

*Etape préliminaire*: préparation de la bis(2,3-dihydroxypropyl)-n-butylamine.

A une solution de 53 g (1,32 mole) d'hydroxyde de sodium dans 600 ml d'éthanol on a ajouté 44 g (0,6 mole) de n-butylamine. Cette solution refroidie à 15°C a été additionnée de 166 g (1,5 moles) de 1-chloro-2,3-propanediol et on a laissé reposer le mélange pendant une nuit. Le chlorure de sodium formé a ensuite été filtré et l'éthanol évaporé à 50°C et sous 2 kPa. Le poids du résidu liquide constitué de bis(2,3-dihydroxypropyl)-n-butylamine brute a été de 161,4 g.

*1ère étape*: préparation du cholorure de n-butyl-tris(2,3-dihydroxypropyl)-ammonium.

Une nouvelle quantité de 66,3 g (0,6 mole) de 1-chloro-2,3-propanediol a été ajoutée à la bis(2,3-dihydroxypropyl)-n-butylamine non purifiée. Ces réactifs ont alors été portés à 100°C pendant 26 heures. La séparation du sel d'ammonium formé à été réalisée en milieu méthanolique à l'aide d'une résine échangeuse de cations (Bio-Rad AG 50W—X8). On a ainsi obtenu 21,9 g de chlorure de n-butyl-tris (2,3-dihydroxypropyl)-ammonium.

*2ème étape*: acétylation du chlorure de n-butyl-tris(2,3-dihydroxypropyl)-ammonium.

Au solide obtenu à l'étape précédente on a ajouté 30,9 g (0,4 mole) de chlorure d'acétyle. Le mélange résultant a été maintenu au reflux pendant 5 heures, soit le temps nécessaire au dégagement de 95% de la quantité théorique d'acide chlorhydrique. Le produit a ensuite été séché sous vide pendant une nuit (25°C—133 Pa) puis recristallisé avec un mélange de tétrahydrofurane et d'éther éthylique (rapport volumique THF/éther = 4:6) avant d'être séché une nouvelle fois à 70°C et sous 133 Pa. On a ainsi obtenu 3,6 g de chlorure de n-butyl-tris(2,3 diacétoxypropyl)-ammonium de pureté supérieure à 95%.

Exemple 4

Préparation du chlorure de méthyl-tris(2-octyloxyéthyl)-ammonium.

*1ère étape*: préparation de la tris(2-octyloxyéthyl)-amine.

A une quantité de 92,9 g (0,62 mole) de triéthanolamine, on a ajouté 310 g (1,87 moles) de chlorure d'octanoyle. Le mélange a été maintenu à 110°C pendant 7 heures durant lesquelles il s'est dégagé, 1,05 moles d'acide chlorhydrique. Le chlorhydrate de tris (2-octyloxyéthyl)-amine formé a été neutralisé par une quantité équivalente d'hydroxyde de sodium en solution dans le méthanol. Le résidu de chorure de sodium a ensuité été filtré et le filtrat évaporé à 50°C, sous une pression de 2 kPa. On a ainsi recueilli 295 g de tris(2-octyloxyéthyl)-amine.

*2ème étape*: quaternisation de la tris(2-octyloxyéthyl)-amine.

120 g de tris(2-octyloxyéthyl)-amine ont été traités par 80,8 g (1,6 moles) de chlorure de méthyle à 90°C en autoclave en présence de 150 ml d'acétonitrile comme solvant. Après 20 heures de réaction, le mélange a été évaporé à 50°C sous une pression de 2 kPa. On a obtenu 114 g de solide brun qui a été lavé plusieurs fois au moyen d'éther de pétrole (fraction 40—60). Après séchage, on a obtenu 56,8 g de chlorure de méthyl-tris(2-octyloxyéthyl)-ammonium de pureté supérieure à 98%.

**Revendications**

1. Composés organiques d'ammonium quaternaire, caractérisés en ce qu'ils sont de formule générale:

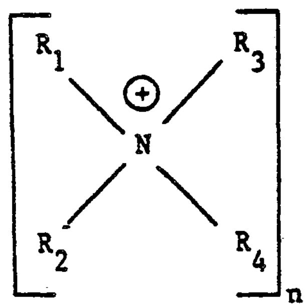

$mA^{\ominus}$

dans laquelle:
$R_1$ représente un groupe alkyle, aryle, arylalkyle ou alkylaryle, non substitué ou substitué par au moins deux groupements ester;
$R_2$, $R_3$ et $R_4$ représentent des groupes alkyle, aryle, arylalkyle ou alkylaryle substitués chacun par au moins un groupement ester de formule:

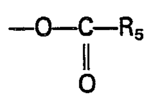

où $R_5$ représente un groupe alkyle, aryle, alkylaryle ou arylalkyle de 1 à 9 atomes de carbone;

$A^{\ominus}$ représente un anion organique ou inorganique;
n et r sont des nombres entiers;
m est un nombre entier valant 1, 2 ou 3 et tel que le produit m x r = n.

2. Composés selon la revendication 1, caractérisés en ce que l'un au moins des groupements ester est un groupement ester aliphatique à chaîne droite ou ramifiée dont le groupe $R_5$ comprend 7 à 9 atomes de carbone.

3. Composés selon la revendication 1, caractérisés en ce que l'un au moins des groupements ester est un groupement ester aliphatique à chaîne droite ou ramifiée dont le groupe $R_5$ comprend moins de 7 atomes de carbone.

4. Composés selon la revendication 3, caractérisés en ce que l'un au moins des groupements ester est un groupement ester acétique.

5. Composés selon la revendication 4, caractérisés en ce qu'ils sont des sels de méthyl-tris(2-acétoxyéthyl)-ammonium.

6. Composés selon la revendication 5, caractérisés en ce qu'ils sont le méthosulfate, le dodécylsulfate, le dodécylbenzènesulfonate et le chlorure de méthyl-tris(2-acétoxyéthyl)-ammonium.

7. Composés selon la revendication 4, caractérisés en ce qu'ils sont constitués par le chlorure de tris(2-acétoxyéthyl)-(2,3-diacétoxypropyl)-ammonium et le chlorure de n-butyl-tris (2,3-diacétoxypropyl)-ammonium.

8. Procédé de préparation de composés conformes à l'une quelconque des revendications 1 à 7, caractérisé en ce que l'on traite une amine tertiaire comprenant un groupement alcool sur chaque groupe lié à l'azote, avec un agent d'alkylation ou d'arylation et avec un agent d'acylation choisi parmi les anhydrides et les chlorures d'acyle.

9. Procédé selon la revendication 8, caractérisé en ce que l'amine tertiaire est la triéthanolamine.

**Patentansprüche**

1. Organische quartäre Ammonium-Verbindungen, gekennzeichnet durch die allgemeine Formel:

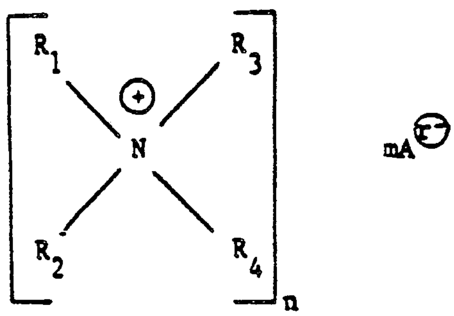

in der

$R_1$ eine nicht-substituierte oder durch wenigstens zwei Ester-Gruppierungen substituierte Alkyl-, Aryl-, Arylalkyl oder Alkylaryl-Gruppe darstellt;

$R_2$, $R_3$ und $R_4$ Alkyl-, Aryl-, Arylalkyl- oder Alkylaryl-Gruppen darstellen, die jede durch wenigstens eine Ester-Gruppierrung der Formel:

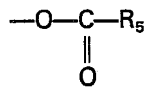

substituiert sind, in der

$R_5$ eine Alkyl-, Aryl-, Alkylaryl oder Arylalkyl-Gruppe mit 1 bis 9 Kohlenstoffatomen darstellt;

$A^{\ominus}$ ein organisches oder anorganisches Anion darstellt;

n und r ganze Zahlen sind

m eine ganze Zahl 1, 2 oder 3 und derart ist, daß das Produkt m x r = n.

2. Zusammensetzungen nach Anspruch 1, dadurch gekennzeichnet, daß wenigstens eine der Ester-Gruppierungen eine gerad- oder verzweigtkettige aliphatische Ester-Gruppierung ist, deren $R_5$—Gruppe 7 bis 9 Kohlenstoffatome umfaßt.

3. Zusammensetzungen nach Anspruch 1, dadurch gekennzeichnet, daß wenigstens eine der Ester-Gruppierungen eine gerad- oder verzweigtkettige aliphatische Ester-Gruppierung ist, deren $R_5$—Gruppe weniger als 7 Kohlennstoffatome umfaßt.

4. Zusammensetzungen nach Anspruch 3, dadurch gekennzeichnet, daß wenigstens eine der Ester-Gruppierungen eine Essigsäure-Ester-Gruppierung ist.

5. Zusammensetzungen nach Anspruch 4, dadurch gekennzeichnet, daß sie Methyl-tris(2-acetoxyethyl)-Ammoniumsalze sind.

6. Zusammensetzungen nach Anspruch 5, dadurch gekennzeichnet, daß sie Methyl-tris(2-acetoxyethyl)-ammonium-methosulfat, -dodecylsulfat, -dodecylbenzolsulfonat und -chlorid sind.

7. Zusammensetzungen nach Anspruch 4, dadurch gekennzeichnet, daß sie durch Tris(2-acetoxyethyl)-(2,3-diacetoxypropyl)-ammoniumchlorid und n-Butyl-tris(2,3-diacetoxypropyl)-ammoniumchlorid gebildet sind.

8. Verfahren zur Herstellung von Zusammensetzungen nach einem der Ansprüche 1 bis 7, dadurch gekennzeichnet, daß ein tertiäres Amin, das eine Alkohol-Gruppeirung an jeder mit dem Stickstoff verbundenen Gruppe umfaßt, mit einem Alkylierungs- oder Arylierungsmittel und einem Acylierungsmittel, ausgewählt unter den Acyhlanhydriden und -chloriden, behandelt wird.

9. Verfahren nach Anspruch 8, dadurch gekennzeichnet, daß das tertiäre Amin Triethanolamin ist.

**Claims**

1. Organmic quaternary ammonium compositions characterized by the general formula:

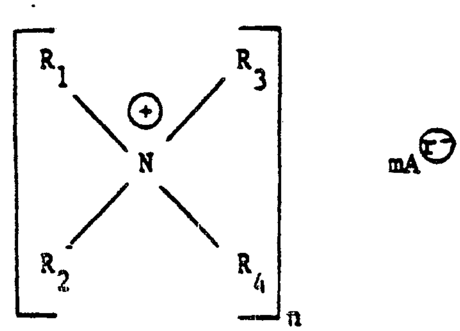

wherein

$R_1$ represents an alkyl, aryl, arylalkyl or alkylaryl group non-substituted or substituted by at least two ester groups;

$R_2$, $R_3$ and $R_4$ represent alkyl, aryl, arylalkyl or alkylaryl groups each substituted by at least one ester group of the formula:

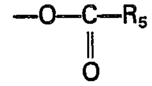

in which $R_5$ represents an alkyl, aryl, alkylaryl or arylalkyl group of 1 to 9 carbon atoms;

$A^{\ominus}$ represents an organic or inorganic anion;
n and r are integers;
m is an integer 1, 2 or 3 and such that the product m x r = n.

2. Compositions according to claim 1, characterized in that at least one of the ester groups is a straight or branched chain aliphatic ester group with the $R_5$ group comprising 7 to 9 carbon atoms.

3. Compositions according to claim 1, characterized in that at least one of the ester groups is a straight or branched chain aliphatic ester group with the $R_5$ group comprising less than 7 carbon atoms.

4. Compositions according to claim 3, characterized in that at least one of the ester groups is an acetic ester group.

5. Compositions according to claim 4, characterized in that they are methyl-tris(2-acetoxyethyl)-ammonium salts.

6. Compositions according to claim 5, characterized in that they are the methosulphate, dodecylsulphate, dodecylbenzenesulphonate and chloride of methyl-tris(2-acetoxyethyl)-ammonium.

7. Compositions according to claim 4, characterized in that they are constituted by the tris(2-acetoxyethyl)-(2,3-diacetoxypropyl)-ammonium-chloride and the n-butyl-tris(2,3-diacetoxyapropyl)-ammonium-chloride.

8. Process for the production of compositions according to one of claims 1 to 7, characterized in that a tertiary amine comprising an alcohol group at each group bound to the nitrogen is treated with an alkylation or arylation agent and with an acylation agent selected from the acylanhydrides and chlorides.

9. Process according to claim 8, characterized in that the tertiary amine is triethanolamine.